(19) Europäisches Patentamt / European Patent Office / Office européen des brevets

(11) **EP 3 614 160 B1**

(12) **EUROPEAN PATENT SPECIFICATION**

(45) Date of publication and mention
of the grant of the patent:
**27.09.2023 Bulletin 2023/39**

(21) Application number: **18189940.2**

(22) Date of filing: **21.08.2018**

(51) International Patent Classification (IPC):
**G01R 33/38** *(2006.01)*    G01R 33/381 *(2006.01)*
**G01R 33/44** *(2006.01)*

(52) Cooperative Patent Classification (CPC):
**G01R 33/3802;** G01R 33/381; G01R 33/445

(54) **METHOD OF RAMPING A MAGNET OF A MAGNETIC RESONANCE IMAGING APPARATUS BASED ON A MEASURED CENTRE FREQUENCY OF THE BODY COIL**

VERFAHREN ZUM HOCHFAHREN EINES MAGNETEN EINES MAGNETRESONANZBILDGEBUNGSAPPARATS BASIEREND AUF EINER GEMESSENEN MITTENFREQUENZ DER KÖRPERSPULE

PROCÉDÉ POUR RALENTIR UN AIMANT D'UN APPAREIL D'IMAGERIE PAR RÉSONANCE MAGNÉTIQUE NUCLÉAIRE UTILISANT UNE FRÉQUENCE CENTRALE MESURÉE DE LA BOBINE DE CORPS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.02.2020 Bulletin 2020/09**

(73) Proprietor: **Siemens Healthcare GmbH**
**91052 Erlangen (DE)**

(72) Inventors:
• **Biber, Stephan**
**91056 Erlangen (DE)**

• **Nistler, Jürgen**
**91056 Erlangen (DE)**

(56) References cited:
**WO-A1-2017/203330    US-A1- 2005 111 159**

• **BEN PARKINSON: "Design considerations and experimental results for MRI systems using HTS magnets", SUPERCONDUCTOR SCIENCE AND TECHNOLOGY, IOP PUBLISHING, TECHNO HOUSE, BRISTOL, GB, vol. 30, no. 1, 16 November 2016 (2016-11-16), page 14009, XP020311900, ISSN: 0953-2048, DOI: 10.1088/0953-2048/30/1/014009 [retrieved on 2016-11-16]**

## Description

The invention describes methods of operating an MRI apparatus. The invention further MRI apparatus, a computer program product and a computer readable medium. Developments in the field of magnetic resonance imaging (MRI) systems have led to advances in low-field systems, which can be preferred on account of their smaller footprint. Low-field systems can be open-bore, can allow interventional procedures, and are less expensive. The term "low-field system" generally refers to a system that has a magnetic field strength of at most 1.0 Tesla. An MRI system with a magnetic field strength in excess of 1.0 Tesla is generally referred to as a "high-field system". The magnetic field strength of the low-field systems currently in development may be even lower than 1.0 Tesla, and may even be lower than 0.5 Tesla.

When an MRI system is first installed on site, a ramping procedure is carried out to set up the main magnetic field (also referred to as the static background field) in the main coil windings. After the initial installation ramping procedure, shim coils are used to perform any adjustments necessary to take into account the local environment. Usually, the target frequency (i.e. the centre frequency of the main magnet) is determined with the aid of a probe placed at a suitable position in the apparatus. To allow for the inevitable decay of the magnetic field arising from component aging, typically in the order of several hundred ppm per year, this target frequency generally exceeds the centre frequency of the body coil by an amount that is sufficient to ensure that the centre frequency of the main magnetic field remains above the body coil centre frequency for as long as possible.

In any superconducting MRI system, decay of the magnetic field is unavoidable due to the residual electrical resistance of the magnet. Field decay means that the centre frequency of the main magnetic field gradually drifts away from the initial setting. Ultimately, it becomes necessary to re-ramp the system. In a high-field system, the bandwidth of the body coil and radio-frequency system is so large (e.g. body coil bandwidth $\pm$ 100 kHz or more) that it can take several years before field decay is out of specification. Generally, another service procedure such as a cold head exchange must be scheduled sooner. To carry out such a service procedure, the magnet must in any case be ramped down and up, so an opportunity is given to re-calibrate the system.

Before ramping the magnet, a target frequency for the main magnetic field is identified. It is usual to set the highest possible target frequency and to use the shim coils for any fine-tuning of the main magnetic field. By setting the highest possible target frequency, the decay window (the time taken for the frequency to drift to the lower end of the allowed band) is made as long as possible. This approach is suitable for systems with a high bandwidth as explained above. However, the bandwidth of a low-field system is significantly narrower than that of a high-field system, being only in the order of 10 kHz - 25 kHz, so that prior art methods of setting the magnet target frequency are limited to a much shorter "decay window". Because the decay window is narrower, the magnet frequency of a low-field MRI system decays towards an out-of-spec level in a shorter space of time. This means that low-field MRI systems must generally be ramped more often.

The first superconducting low-field MRI systems were generally realised as vertical field systems, characterized by a relatively inefficient body coil. These early low-field vertical systems are known to be less reliable than a comparable horizontal field system (with a birdcage body coil), so that service checks must be scheduled relatively frequently. During these service procedures, the frequency of the main magnetic field is checked and the system is re-ramped if necessary. In a low-field system, it may be necessary to carry out an intermittent ramping procedure, for example in the event of an infrastructure problem such as a loss of power or problems with a cooling issues arrangement, etc.

Ideally, the centre frequency of the body coil would be the same as the frequency of the main magnet field. However, with a low-field magnet, reflection effects reduce the accuracy of the system and the centre frequency is lower.

The narrow bandwidth of the radio-frequency system, especially the body coil and the receiver coils, means that reflection coefficients significantly reduce the available power from the Radio Frequency Power Amplifier (RFPA). When the reflection coefficients from the body coil are not equal, power is partly reflected back into the RFPA, which leads to a derating of the RFPA. This reduces the available power for the radio frequency magnetic field generated by the transmit coil or body coil. This magnetic field is generally referred to as the B1 field. Furthermore, the centre frequency of the magnet and the centre frequency of the body coil are not necessarily identical. For these reasons, the magnet centre frequency must be greater than the body coil centre frequency to allow for inevitable main magnet field decay over time. However, it is difficult to identify a magnet target frequency that works satisfactorily within the constraints of narrow body coil bandwidth and high reflection coefficients.

The document WO 2017/203330 A1 discloses systems and methods for magnetic resonance imaging of multiple different nuclear spin species using the same radio frequency coil. Generally, multiple different nuclear spin species are imaged using the same RF coil by using an MRI system whose magnetic field can be rapidly ramped between a number of different, and arbitrary, magnetic field strengths. The magnetic field of this MRI system can be ramped to different values in reasonable amounts of time.

The object of the invention is to provide a way of ramping an MRI apparatus that overcomes the problems outlined above.

This object is achieved by the invention which is defined by the methods of claims 1 and 2 of operating an MRI apparatus, by the MRI apparatus of claims 8 and 9, by a computer program product according to claim 14, and by a computer-readable medium according to claim 15.

The invention relates to a method of operating an MRI apparatus comprising a magnet, which method comprises the steps of - exciting the body coil (BC) of the MRI apparatus to emit a radio-frequency signal (fBC ); - detecting a reflected radio-frequency signal (fBc '), that is reflected by the body coil (BC); - determining the centre frequency (fc ) of a resonance curve of the body coil (BC) from the reflected radio-frequency signal (fBc ') by measuring the reflection coefficients of the body coil (BC) as a function of frequency; - identifying a magnet target frequency (fT ) on the basis of the determined centre frequency (fc ); and - ramping the magnet to the magnet target frequency (fT ).

An alternative method according to the invention relates to s method of operating an MRI apparatus comprising a magnet, a body coil and a further coil, which method comprises the steps of - exciting the body coil (BC) of the MRI apparatus to emit a radio-frequency signal (fBC ); - detecting the emitted radio-frequency signal (fBc ') using the further coil; - determining the centre frequency (fc ) of a resonance curve of the body coil (BC) from the detected radio-frequency signal (fBc ') by measuring the transmissivity between the body coil (BC) and the further coil; - identifying a magnet target frequency (fT ) on the basis of the determined centre frequency (fc ); and - ramping the magnet to the magnet target frequency (f T ) .

The magnet is ramped at a scheduled time, whereby this is to be understood to mean that the magnet field is monitored continually during operation of the MRI apparatus, and when it is detected that the magnet field has decayed to a level approaching a lower limit, an alert may be issued to an operator who can then schedule a ramp procedure at the next convenient opportunity.

An advantage of the invention is that the centre frequency of the body coil resonance curve is empirically determined and used as a basis for computing the magnet target frequency. This is advantageous especially in low-field systems for which the body coil bandwidth is relatively narrow. A further advantage is that very little additional effort is required to implement the inventive method. Instead of providing a means to tune the body coil and another means to tune the magnet, the invention takes the approach of using the body coil as it is, and tuning the magnet on the basis of the body coil. Any drift of the body coil centre frequency (for example as a result of aging, from varying mechanical load on the patient table of the body coil, etc.), will be taken into consideration in the target frequency for the magnet. In other words, the magnet will always be ramped to suit the momentary state of the body coil. It shall be understood that the body coil will be tuned to within a specified frequency bandwidth.

The invention further relates to an MRI apparatus with a magnet comprising

- a body coil excitation unit for exciting a body coil (BC) to emit a radio-frequency signal (fBC);
- a frequency computation module realised to determine the centre frequency (fc) of a resonance curve of the reflected radio-frequency signal (fB c'), that is reflected by the body coil (BC), by measuring the reflection coefficients of the body coil (BC) as a function of frequency;
- a target frequency computation unit realised to calculate a magnet target frequency (fT) on the basis of the determined centre frequency (fC); and - a magnet power supply unit realised to ramp the magnet to the target frequency (fT ).

In an alternative arrangement, the invention relates to an MRI apparatus with a magnet comprising

- a body coil excitation unit for exciting a body coil (BC) to emit a radio-frequency signal (fBC);
- a further coil; - a frequency computation module realised to determine the centre frequency (fc) of a resonance curve of the emitted radio-frequency signal, that is detected by the further coil, by measuring the transmissivity between the body coil (BC) and the further coil;
- a target frequency computation unit realised to calculate a magnet target frequency (fT) on the basis of the determined centre frequency (fc); and
- a magnet power supply unit realised to ramp the magnet to the target frequency (fT).

Another advantage of the inventive MRI apparatus is that they can be significantly more economical to run than a comparable prior art MRI apparatus, since the time interval between consecutive ramping events can be extended, resulting in less downtime (during which MRI scans cannot be performed).

The units or modules of the MRI apparatus mentioned above, in particular the frequency determination unit and the selection unit, can be completely or partially realised as software modules running on a processor of a control unit of an MRI apparatus. A realisation largely in the form of software modules can have the advantage that applications already installed on an existing MRI system can be updated, with relatively little effort, to carry out the method steps of the present application. The object of the invention is also achieved by a computer program product according to claim 14. In addition to the computer program, such a computer program product can also comprise further parts such as documentation and/or additional components, also hardware components such as a hardware key (dongle etc.) to facilitate access to the software.

The invention also relates to a computer readable medium according to claim 15. This computer readable medium can be a medium such as a memory stick, a hard-disk or other transportable or permanently-installed carrier can serve to transport and/or to store the executable parts of the computer program product so that these can be read from a processor unit of an MRI apparatus. A processor unit can comprise one or more microprocessors or their equivalents.

Particularly advantageous embodiments and features of the invention are given by the dependent claims, as revealed in the following description.

The inventive method can be applied in any appropriate MRI apparatus, for example in an MRI apparatus that has a superconducting magnet, a permanent magnet, or an electromagnet. However, a low-field MRI apparatus with a superconducting magnet benefits to a greater extent from the inventive method. In the following therefore, but without restricting the invention in any way, it may be assumed that the magnet of the MRI apparatus is a superconductive magnet. It may also be assumed that the MRI apparatus is a low-field MRI apparatus. In a particularly preferred embodiment of the invention, the field strength of the magnet is at most 1.0 T, more preferably at most 0.7 T, most preferably at most 0.5 T. As indicated above, the RF bandwidth of a mid- or high-field MRI apparatus is generally large (e.g. body coil bandwidth $\pm$ 100 kHz or more), but the bandwidth of a low-field apparatus is significantly narrower. In a further preferred embodiment of the invention, the MRI apparatus has a body coil with a RF bandwidth that does not exceed 50 kHz.

An advantage of the invention is that the target frequency of the magnet can be determined independently of an imaging sequence or MR-experiment (which would of course require the main magnetic field B0), and is determined solely on the basis of reflection parameters of the body coil. In a particularly preferred embodiment of the invention, the magnet target frequency is determined in the absence of a main magnetic field. In other words, the magnet target frequency can be determined even when the main magnet has been ramped down. The invention overcomes a problem associated with low-field MRI systems, namely that in such low-field systems, it is no longer practicable to specify the magnet frequency independently of the body coil frequency. In contrast to a high-field systems with a body coil bandwidth large enough to include a separately specified magnet frequency, the bandwidth of a low-field system is so narrow that the prior art approach of independently specifying the magnet frequency runs the risk of not lying within the body coil bandwidth.

To determine a suitable target frequency for the magnet in the next ramp event, the body coil centre frequency is determined by measuring the reflection coefficients of the body coil as a function of frequency. To this end, a body coil excitation unit excites the body coil to emit an RF signal at a set or chosen frequency. According to the invention, the centre frequency of the body coil is deduced from a signal reflected by the body coil. The resonance curve of the reflected signal is detected and averaged in a suitable signal processing module to determine its centre frequency. These computations are preferably performed in the frequency domain.

The resonance curve can be established by using a directional coupler to measure the reflectivity of the body coil. Such a directional coupler may already be a component of the MRI system. However, some simple MRI systems may not include such a directional coupler, or a directional coupler may be present but not equipped with the necessary detectors. Therefore, in a further preferred embodiment of the invention, the centre frequency of the reflected signal is determined by measuring the transmissivity between the body coil and a further coil of the MRI apparatus. This can be achieved in a relatively straightforward manner by using a wide-band transmit antenna to transmit a signal, and by detecting the received signal. In one approach, this further coil can be a local coil of the MRI apparatus. Alternatively, the further coil can be a pickup coil of the MRI apparatus. Once the body coil centre frequency has been determined, the magnet target frequency can be computed. In a particularly preferred embodiment of the invention, the magnet target frequency is adjusted by adding an offset to the determined body coil centre frequency.

Other objects and features of the present invention will become apparent from the following detailed descriptions considered in conjunction with the accompanying drawings. It is to be understood, however, that the drawings are designed solely for the purposes of illustration and not as a definition of the limits of the invention.

Fig 1 shows a simplified circuit diagram of a superconductive low-field MRI apparatus according to an embodiment;

Fig 2 shows a simplified block diagram of an MRI apparatus according to an embodiment;

Fig 3 illustrates the determination of a magnet target frequency using the inventive methods.

In the diagrams, like numbers refer to like objects throughout. Objects in the diagrams are not necessarily drawn to scale.

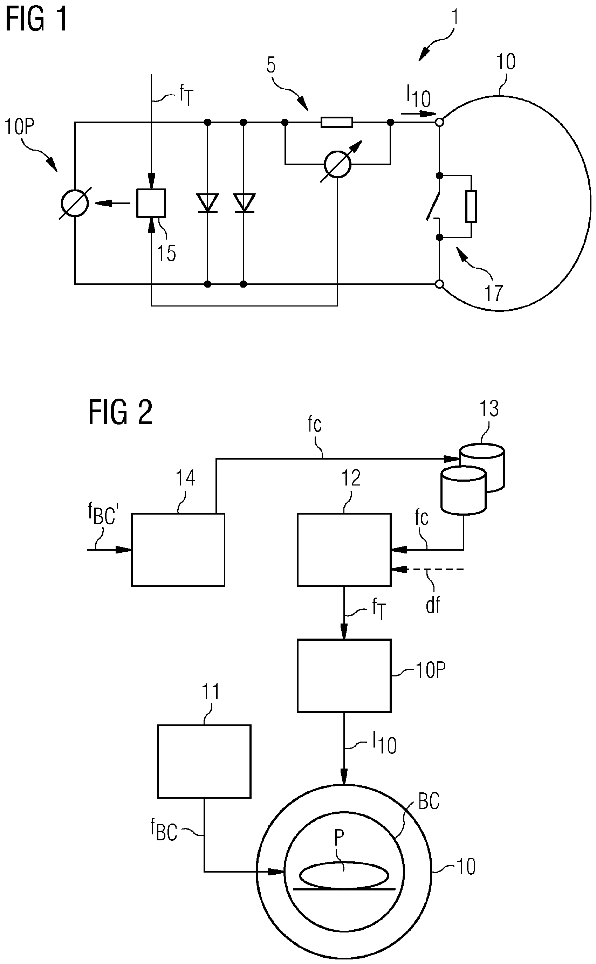

Fig 1 shows a greatly simplified circuit diagram of a superconductive low-field MRI apparatus 1. An MRI system 1 comprises various modules and units, most of which will be known to the skilled person and need not be explained here.

The apparatus comprises a main magnet 10 that generates a very homogenous main magnetic field B0. An MPSU 10P is used to supply current $I_{10}$ to the magnet 10 during a ramp-up procedure, when the magnet 10 is ramped to a previously determined target frequency. A switch assembly 17 comprising a superconducting switch in parallel with a bypass resistor is shown connected across the main magnet coil. The switch is closed during the ramp-up procedure so that a small amount of current passes through the bypass resistor. In this exemplary embodiment, an ammeter shunt S is used to measure the magnet current $I_{10}$ during ramping so that a power supply controller 15 can estimate the magnet frequency and compare it to the target frequency $f_T$ so that the ramp-up procedure can be halted when the target frequency $f_T$ has been attained. At this point, the switch 17 is opened again.

Fig 2 shows a simplified block diagram of an MRI apparatus 1 indicating the main magnet 10 and the body coil BC. The usual arrangement of additional coils such as shim coils, local coil, pickup coil and a number of gradient coils may be assumed to be present. The diagram shows a body coil excitation unit 11 realised to excite the body coil BC to emit an RF signal at a chosen frequency $f_{BC}$. The reflected RF signal $f_{BC}'$ is detected and the resonance curve of the reflected RF signal $f_{BC}'$ is analysed in frequency determination module 14 to identify its centre frequency $f_c$. The centre frequency $f_c$ is stored in a memory module 13, which can be realised as a memory module of the body coil BC, or as a memory module of a control unit of the MRI apparatus 1. The reflection coefficients of the system mean that the centre frequency $f_c$ of the reflected body coil signal $f_{BC}'$ is lower than the body coil frequency $f_{BC}$.

A target frequency computation module 12 determines a magnet target frequency $f_T$ on the basis of the identified centre frequency $f_c$. Depending on the type of ramp-up sequence that is to be carried out, an offset df may be added to the frequency $f_c$. In an exemplary process flow, the centre frequency $f_c$ can be identified for example by the manufacturer or at some point during the lifetime of the MRI apparatus. Either way, the centre frequency $f_c$ is stored in the memory module 13. Before carrying out a ramp-up sequence, the centre frequency $f_c$ is retrieved from the memory module 13, and adjusted as necessary or as desired by a suitable offset df to give the target frequency $f_T$, and the magnet is ramped to the target frequency $f_T$.

A ramp control module is provided to initiate a subsequent ramping procedure at a suitable time. The magnet power supply unit 10P accordingly supplies current $I_{10}$ to the magnet 10 during the ramp-up procedure in order to ramp the main magnet 10 to that target frequency $f_T$.

The units and modules described above can be realized as part of a central control system of the MRI apparatus 1.

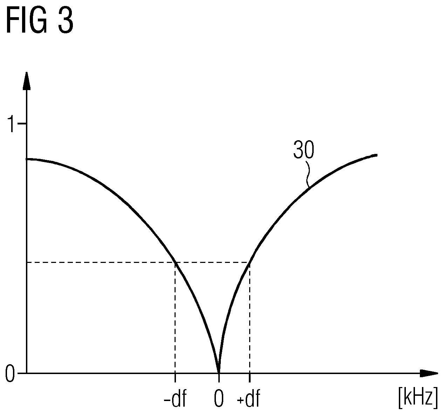

Fig 3 shows an exemplary resonance curve of a body coil. The Y-axis shows reflection coefficients between 0 and 1. The X-axis shows frequency offset in kHz, with 0 corresponding to the minimum reflection coefficient. Such a curve is obtained by averaging the reflected body coil signal. The shape of the resonance curve 30 is determined largely by the Q-factor of the body coil. For the comparatively low magnet frequency of a low-field MRI system, the body coil has a high Q-factor during an imaging sequence (i.e. with a patient inside the body coil) on account of the low ohmic losses arising from lower conductivity. In a low-field MRI system, therefore, the quality of an imaging procedure is dependent on how well the magnet frequency and body coil frequency are matched. The lowest point or minimum of the resonance curve, corresponding to the centre frequency $f_c$ of the reflected body coil signal, is identified and used to arrive at a magnet target frequency for a subsequent ramp-up sequence.

The target frequency can be set as the identified centre frequency $f_c$ that was identified in the resonance curve of the body coil reflection). However, it is preferred to add an offset to the target frequency. The magnitude of the offset can be chosen on the basis of the shape of the resonance curve and/or on various parameters of the ramp-up sequence.

For example, by identifying a maximum reflection coefficient as indicated in Fig 3, a resulting offset df can be identified. Generally, it is desired to set the magnet target frequency to be higher than the body coil frequency.

Therefore, the target frequency $f_T$ can be expressed as

$$f_T = f_c + df$$

Alternatively, a fraction of the offset may be used, for example 250 of the offset. In this case, the target frequency $f_T$ can be expressed as

$$f_T = f_c + \frac{df}{4}$$

To give an example, the centre frequency $f_c$ of the reflected signal may be determined to be 20.0 MHz. Adding a suitable offset such as 50 kHz, the target frequency $f_T$ for the next ramp event is determined to be 20.05 MHz using the above equation. In this way, the target frequency $f_T$ can be identified on the basis of a desired accuracy of the intended ramping procedure. The inventive method of using an echo experiment to determine the magnet target frequency is associated with a favourably high degree of accuracy, i.e. with an error of less than 1.0 kHz. In another example, the centre frequency $f_c$ of the reflected signal may be determined to be 30.1 MHz. Adding a suitable offset such as 10 kHz, the target frequency $f_T$ for the next ramp event is determined to be 30.11 MHz using the above equation.

Excitation of the body coil BC, measurement of the reflected body coil signal $f_{BC}'$ and computation of the centre frequency $f_c$ and the target frequency $f_T$ can be performed entirely independently of the main magnet field B0, so that the inventive method can be carried out when the magnet 10 is ramped down.

Although the present invention has been disclosed in the form of preferred embodiments and variations thereon, it will be understood that numerous additional modifications and variations could be made thereto without departing from the scope of the invention as defined by the claims.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

**Claims**

1. A method of operating an MRI apparatus (1) comprising a magnet (10), which method comprises the steps of

   - exciting the body coil (BC) of the MRI apparatus (1) to emit a radio-frequency signal ($f_{BC}$);
   - detecting a reflected radio-frequency signal ($f_{BC}'$), that is reflected by the body coil (BC);
   - determining the centre frequency ($f_c$) of a resonance curve (30) of the body coil (BC) from the reflected radio-frequency signal ($f_{BC}'$) by measuring the reflection coefficients of the body coil (BC) as a function of frequency;
   - identifying a magnet target frequency ($f_T$) on the basis of the determined centre frequency ($f_c$); and
   - ramping the magnet (10) to the magnet target frequency ($f_T$).

2. A method of operating an MRI apparatus (1) comprising a magnet (10), a body coil and a further coil, which method comprises the steps of

   - exciting the body coil (BC) of the MRI apparatus (1) to emit a radio-frequency signal ($f_{BC}$);
   - detecting the emitted radio-frequency signal ($f_{BC}'$) using the further coil;
   - determining the centre frequency ($f_c$) of a resonance curve (30) of the body coil (BC) from the detected radio-frequency signal ($f_{BC}'$) by measuring the transmissivity between the body coil (BC) and the further coil;
   - identifying a magnet target frequency ($f_T$) on the basis of the determined centre frequency ($f_c$); and
   - ramping the magnet (10) to the magnet target frequency ($f_T$).

3. A method according to one of claims 1 or 2, comprising a step of storing the centre frequency ($f_c$) in a storage module (13) of the MRI apparatus (1).

4. A method according to one of claim 1 or claim 3 when dependent on claim 1, wherein the centre frequency ($f_c$) is acquired by averaging the resonance curve of the reflected radio-frequency signal ($f_{BC}'$).

5. A method according to any of the preceding claims, wherein the magnet (10) is ramped to a frequency comprising the sum of the magnet target frequency ($f_T$) and an offset (df), wherein the offset (df) is determined on the basis of the ramping procedure.

6. A method according to any of the preceding claims, wherein the centre frequency ($f_c$) of the body coil resonance curve (30) is determined during manufacture of the MRI apparatus (1) and/or at any time during the lifetime of the MRI apparatus (1).

7. A method according to any of the preceding claims, wherein the target frequency ($f_T$) of the magnet (10) is determined in the absence of a main magnetic field.

8. An MRI apparatus (1) with a magnet (10) comprising

   - a body coil excitation unit (11) for exciting a body coil (BC) to emit a radio-frequency signal ($f_{BC}$);
   - a frequency computation module (14) realised to determine the centre frequency ($f_c$) of a resonance curve (30) of the reflected radio-frequency signal ($f_{BC}'$), that is reflected by the body coil (BC), by measuring the reflection coefficients of the body coil (BC) as a function of frequency;
   - a target frequency computation unit (12) realised to calculate a magnet target frequency ($f_T$) on the basis of the determined centre frequency ($f_c$); and
   - a magnet power supply unit (10P) realised to ramp the magnet (10) to the target frequency ($f_T$).

9. An MRI apparatus (1) with a magnet (10) comprising

   - a body coil excitation unit (11) for exciting a body coil (BC) to emit a radio-frequency signal ($f_{BC}$);
   - a further coil;
   - a frequency computation module (14) realised to determine the centre frequency ($f_c$) of a resonance curve (30) of the emitted radio-frequency signal, that is detected by the further coil, by measuring the transmissivity between the body coil (BC) and the further coil;
   - a target frequency computation unit (12) realised to calculate a magnet target frequency ($f_T$) on the basis of the determined centre frequency ($f_c$); and
   - a magnet power supply unit (10P) realised to ramp the magnet (10) to the target frequency ($f_T$).

10. An MRI apparatus according to claim 8 or 9, wherein the magnet (10) of the MRI apparatus (1) is designed to have a field strength of at most 1.0 Tesla, more preferably at most 0.7 Tesla, most preferably at most 0.5 Tesla.

11. An MRI apparatus according to one of claims 8 to 10, wherein the magnet (10) is a superconductive magnet.

12. An MRI apparatus according to any of claims 8 to 11, wherein the bandwidth of the body coil comprises at most 100 kHz, more preferably at most 50 kHz.

13. An MRI apparatus according to any of claims 8 to 12, comprising a storage module (13) for storing the determined centre frequency ($f_c$), which storage module (13) is realised as part of the body coil (BC) and/or in a control unit of the MRI apparatus (1).

14. A computer program product comprising a computer program that is directly loadable into a memory of a control unit of an MRI apparatus (1) according to any of (A) claims 8, 10-13 and which comprises program elements for performing the steps of the method according to any of claims 1, 3-7 when the computer program is executed by the control unit, or (B) claims 9-13 and which comprises program elements for performing the steps of the method according to any of claims 2-7 when the computer program is executed by the control unit.

15. A computer-readable medium on which is stored program elements that can be read and executed by a control unit of an MRI apparatus (1) according to any of (A) claims 8, 10-13 in order to perform the steps of the method of any of claims 1, 3-7 when the program elements are executed by the control unit, or (B) claims 9-13 when the program elements are executed by the control unit in order to perform the steps of the method of any of claims 2-7. when the program elements are executed by the computer unit.

**Patentansprüche**

1. Verfahren zum Betreiben eines MRT-Geräts (1), umfassend einen Magneten (10), wobei das Verfahren die Schritte umfasst:

- Erregen der Körperspule (BC) des MRT-Geräts (1), um ein Hochfrequenzsignal ($f_{BC}$) zu emittieren;
- Detektieren eines reflektierten Hochfrequenzsignals ($f_{BC}'$), das von der Körperspule (BC) reflektiert wird;
- Bestimmen der Mittenfrequenz ($f_c$) einer Resonanzkurve (30) der Körperspule (BC) aus dem reflektierten Hochfrequenzsignal ($f_{BC}'$) durch Messen der Reflexionskoeffizienten der Körperspule (BC) als Funktion der Frequenz;
- Identifizieren einer Magnetzielfrequenz ($f_T$) basierend auf der bestimmten Mittenfrequenz ($f_c$); und
- Hochfahren des Magneten (10) auf die Magnetzielfrequenz ($f_T$).

2. Verfahren zum Betreiben eines MRT-Geräts (1), umfassend einen Magneten (10), eine Körperspule und eine weitere Spule, wobei das Verfahren die Schritte umfasst:

- Erregen der Körperspule (BC) des MRT-Geräts (1), um ein Hochfrequenzsignal ($f_{BC}$) zu emittieren;
- Detektieren des emittierten Hochfrequenzsignals ($f_{BC}'$) unter Verwendung der weiteren Spule;
- Bestimmen der Mittenfrequenz ($f_c$) einer Resonanzkurve (30) der Körperspule (BC) aus dem detektierten Hochfrequenzsignal ($f_{BC}'$) durch Messen der Transmissivität zwischen der Körperspule (BC) und der weiteren Spule;
- Identifizieren einer Magnetzielfrequenz ($f_T$) basierend auf der bestimmten Mittenfrequenz ($f_c$); und
- Hochfahren des Magneten (10) auf die Magnetzielfrequenz ($f_T$).

3. Verfahren nach einem der Ansprüche 1 oder 2, umfassend einen Schritt des Speicherns der Mittenfrequenz (fc) in einem Speichermodul (13) des MRT-Geräts (1).

4. Verfahren nach einem von Anspruch 1 oder Anspruch 3 in Abhängigkeit von Anspruch 1, wobei die Mittenfrequenz (fc) durch Mitteln der Resonanzkurve des reflektierten Hochfrequenzsignals (fBC') akquiriert wird.

5. Verfahren nach einem der vorhergehenden Ansprüche, wobei der Magnet (10) auf eine Frequenz hochgefahren wird, umfassend die Summe aus der Magnetzielfrequenz (fT) und einem Versatz (df), wobei der Versatz basierend auf der Hochfahrprozedur bestimmt wird.

6. Verfahren nach einem der vorhergehenden Ansprüche, wobei die Mittenfrequenz (fc) der Körperspulenresonanzkurve (30) während der Fertigung des MRT-Geräts (1) und/oder zu beliebiger Zeit während der Lebensdauer des MRT-Geräts (1) bestimmt wird.

7. Verfahren nach einem der vorhergehenden Ansprüche, wobei die Zielfrequenz (fT) des Magneten (10) in Abwesenheit eines magnetischen Hauptfeldes bestimmt wird.

8. MRT-Gerät (1) mit einem Magneten (10), umfassend:

   - eine Körperspulenerregungseinheit (11) zum Erregen einer Körperspule (BC), um ein Hochfrequenzsignal ($f_{BC}$) zu emittieren;
   - ein Frequenzberechnungsmodul (14), das realisiert ist, um die Mittenfrequenz ($f_c$) einer Resonanzkurve (30) des reflektierten Hochfrequenzsignals ($f_{BC}$'), das von der Körperspule (BC) reflektiert wird, zu bestimmen, indem die Reflexionskoeffizienten der Körperspule (BC) als Funktion der Frequenz gemessen werden;
   - eine Zielfrequenzberechnungseinheit (12), die realisiert ist, um basierend auf der bestimmten Mittenfrequenz ($f_c$) eine Magnetzielfrequenz ($f_T$) zu berechnen; und
   - eine Magnetleistungsversorgungseinheit (10P), die realisiert ist, um den Magneten (10) auf die Zielfrequenz ($f_T$) hochzufahren.

9. MRT-Gerät (1) mit einem Magneten (10), umfassend:

   - eine Körperspulenerregungseinheit (11) zum Erregen einer Körperspule (BC), um ein Hochfrequenzsignal ($f_{BC}$) zu emittieren;
   - eine weitere Spule;
   - ein Frequenzberechnungsmodul (14), das realisiert ist, um die Mittenfrequenz ($f_c$) einer Resonanzkurve (30) des emittierten Hochfrequenzsignals, die von der weiteren Spule detektiert wird, zu bestimmen, indem die Transmissivität zwischen der Körperspule (BC) und der weiteren Spule gemessen wird;
   - eine Zielfrequenzberechnungseinheit (12), die realisiert ist, um basierend auf der bestimmten Mittenfrequenz ($f_c$) eine Magnetzielfrequenz ($f_T$) zu berechnen; und
   - eine Magnetleistungsversorgungseinheit (10P), die realisiert ist, um den Magneten (10) auf die Zielfrequenz ($f_T$) hochzufahren.

10. MRT-Gerät nach Anspruch 8 oder 9, wobei der Magnet (10) des MRT-Geräts (1) so konzipiert ist, dass er eine Feldstärke von höchstens 1,0 Tesla, bevorzugter höchstens 0,7 Tesla, am meisten bevorzugt höchstens 0,5 Tesla hat.

11. MRT-Gerät nach einem der Ansprüche 8 bis 10, wobei der Magnet (10) ein supraleitender Magnet ist.

12. MRT-Gerät nach einem der Ansprüche 8 bis 11, wobei die Bandbreite der Körperspule höchstens 100 kHz, bevorzugter höchstens 50 kHz umfasst.

13. MRT-Gerät nach einem der Ansprüche 8 bis 12, umfassend ein Speichermodul (13) zum Speichern der bestimmten Mittenfrequenz (fc), wobei das Speichermodul (13) als Teil der Körperspule (BC) und/oder in einer Steuereinheit des MRT-Geräts (1) realisiert ist.

14. Computerprogrammprodukt, umfassend ein Computerprogramm, das direkt ladbar ist in einen Speicher einer Steuereinheit eines MRT-Geräts (1) gemäß einem von (A) den Ansprüchen 8, 10 bis 13, und das Programmelemente zum Durchführen der Schritte des Verfahrens gemäß einem der Ansprüche 1, 3 bis 7 umfasst, wenn das Computerprogramm durch die Steuereinheit ausgeführt wird, oder (B) den Ansprüchen 9 bis 13, und das Programmelemente zum Durchführen der Schritte des Verfahrens nach einem der Ansprüche 2 bis 7 umfasst, wenn das Computerprogramm durch die Steuereinheit ausgeführt wird.

15. Computerlesbares Medium, auf dem Programmelemente gespeichert sind, die gelesen und ausgeführt werden können durch eine Steuereinheit eines MRT-Geräts (1) gemäß einem von (A) den Ansprüchen 8, 10 bis 13, um die Schritte des Verfahrens nach einem der Ansprüche 1, 3 bis 7 durchzuführen, wenn die Programmelemente durch die Steuereinheit ausgeführt werden, oder (B) den Ansprüchen 9 bis 13, wenn die Programmelemente durch die Steuereinheit ausgeführt werden, um die Schritte des Verfahrens nach einem der Ansprüche 2 bis 7 durchzuführen, wenn die Programmelemente durch die Computereinheit ausgeführt werden.

**Revendications**

1. Procédé pour faire fonctionner une installation (1) d'imagerie par résonance magnétique comprenant un aimant (10), lequel procédé comprend les stades de

   - excitation de la bobine (BC) de corps de l'installation (1) d'imagerie par résonance magnétique pour émettre un signal ($f_{BC}$) de fréquence radio ;
   - détection d'un signal ($f_{BC}$') réfléchi de fréquence radio, qui est réfléchi par la bobine (BC) de corps ;
   - détermination de la fréquence ($f_c$) centrale d'une courbe (30) de résonance de la bobine (BC) de corps, à partir du signal ($f_{BC}$') réfléchi de fréquence radio, en mesurant les coefficients de réflexion de la bobine (BC) de corps en fonction de la fréquence ;

- identification d'une fréquence ($f_T$) d'aimant cible sur la base de la fréquence ($f_c$) centrale déterminée ; et
- amenée de l'aimant (10) à la fréquence ($f_T$) d'aimant cible.

2. Procédé pour faire fonctionner de l'installation (1) d'imagerie par résonnance magnétique comprenant un aimant (10), une bobine de corps et une autre bobine, lequel procédé comprend les stades de

- excitation de la bobine (BC) de corps d'une installation (1) d'imagerie par résonnance magnétique pour émettre un signal ($f_{BC}$) de fréquence radio ;
- détection d'un signal ($f_{BC}'$) réfléchi de fréquence radio, qui est réfléchi par l'autre bobine ;
- détermination de la fréquence ($f_c$) centrale d'une courbe (30) de résonnance de la bobine (BC) de corps, à partir du signal ($f_{BC}'$) détecté de fréquence radio, en mesurant la transmitivité entre la bobine (BC) de corps et l'autre bobine ;
- identification d'une fréquence ($f_T$) d'aimant cible sur la base de la fréquence ($f_c$) centrale déterminée ; et
- amenée de l'aimant (10) à la fréquence ($f_T$) d'aimant cible.

3. Procédé suivant l'une des revendications 1 ou 2, comprenant un stade de mise en mémoire de la fréquence ($f_c$) centrale dans un module (13) de mémoire de l'installation (1) d'imagerie par résonnance magnétique.

4. Procédé suivant la revendication 1 ou la revendication 3 lorsqu'elle dépend de la revendication 1, dans lequel on acquiert la fréquence ($f_c$) centrale en faisant la moyenne de la courbe de résonnance du signal ($f_{BC}'$) réfléchi de fréquence radio.

5. Procédé suivant l'une quelconque des revendications précédentes, dans lequel on amène l'aimant (10) à une fréquence comprenant la somme de la fréquence ($f_T$) d'aimant cible et d'un décalage (df), dans lequel le décalage (df) est déterminé sur la base de la procédure d'amenée.

6. Procédé suivant l'une quelconque des revendications précédentes, dans lequel on détermine la fréquence ($f_c$) centrale de la courbe (30) de résonnance de la bobine de corps pendant la fabrication de l'installation (1) d'imagerie par résonnance magnétique et/ou à n'importe quel moment pendant la durée de vie de l'installation (1) d'imagerie par résonnance magnétique.

7. Procédé suivant l'une quelconque des revendications précédentes, dans lequel on détermine la fréquence ($f_T$) cible de l'aimant (10) en l'absence d'un champ magnétique principal.

8. Installation (1) d'imagerie par résonnance magnétique ayant un aimant (10) comprenant

- une unité (11) d'excitation d'une bobine de corps pour exciter une bobine (BC) de corps afin d'émettre un signal ($f_{BC}$) de fréquence radio ;
- un module (14) de calcul de fréquence réalisé pour déterminer la fréquence ($f_c$) centrale d'une courbe (30) de résonnance du signal ($f_{BC}'$) réfléchi de fréquence radio, qui est réfléchi par la bobine (BC) de corps, en mesurant les coefficients de réflexion de la bobine (BC) de corps en fonction de la fréquence ;
- une unité (12) de calcul de la fréquence cible réalisée pour calculer une fréquence ($f_T$) d'aimant cible sur la base de la fréquence ($f_c$) centrale déterminée ; et
- une unité (10P) d'alimentation en puissance de l'aimant réalisée pour amener l'aimant (10) à la fréquence ($f_T$) cible.

9. Installation (1) d'imagerie par résonnance magnétique ayant un aimant (10) comprenant

- une unité (11) d'excitation d'une bobine de corps pour exciter une bobine (BC) de corps afin d'émettre un signal ($f_{BC}$) de fréquence radio ;
- une autre bobine ;
- un module (14) de calcul de la fréquence réalisé pour déterminer la fréquence ($f_c$) centrale d'une courbe (30) de résonnance du signal émis de fréquence radio, qui est détecté par l'autre bobine en mesurant la transmitivité entre la bobine (BC) de corps et l'autre bobine ;
- une unité (12) de calcul de la fréquence cible réalisée pour calculer une fréquence ($f_T$) d'aimant cible sur la base de la fréquence ($f_c$) centrale déterminée ; et
- une unité (10P) d'alimentation en puissance d'un aimant réalisée pour amener l'aimant (10) à la fréquence ($f_T$) cible.

10. Installation d'imagerie par résonnance magnétique suivant la revendication 8 ou 9, dans laquelle l'aimant (10) de l'installation (1) d'imagerie par résonnance magnétique est conçu pour avoir une intensité de champ d'au plus 1,0 tesla, d'une manière plus préférée d'au plus 0,7 tesla, d'une manière encore plus préférée d'au plus 0,5 tesla.

11. Installation d'imagerie par résonnance magnétique suivant l'une des revendications 8 à 10, dans lequel l'aimant (10) est un aimant supraconducteur.

12. Installation d'imagerie par résonnance magnétique suivant l'une quelconque des revendications 8 à 11, dans laquelle la largeur de bande de la bobine de corps est au plus de 100 kHz, d'une façon plus préférée au plus de 50 kHz.

**13.** Installation d'imagerie par résonnance magnétique suivant l'une quelconque des revendications 8 à 12, comprenant un module (13) de mémoire pour mettre en mémoire la fréquence ($f_c$) centrale déterminée, lequel module (13) de mémoire est réalisé comme partie de la bobine (BC) de corps et/ou dans une unité de commande de l'installation (1) d'imagerie par résonnance magnétique.

**14.** Produit de programme d'ordinateur comprenant un programme d'ordinateur, qui peut être chargé directement dans une mémoire d'une unité de commande d'une installation (1) d'imagerie par résonnance magnétique suivant l'une quelconque de (A) des revendications 8, 10 à 13, et qui comprend des éléments de programme pour effectuer les stades du procédé suivant l'une quelconque des revendications 1, 3 à 7, lorsque le programme d'ordinateur est exécuté par l'unité de commande, ou (B) des revendications 9 à 13, et qui comprend des éléments de programme pour effectuer les stades du procédé suivant l'une quelconque des revendications 2 à 7, lorsque le programme d'ordinateur est exécuté par l'unité de commande.

**15.** Support, déchiffrable par ordinateur, sur lequel sont mémorisés des éléments de programme, qui peuvent être déchiffrés et exécutés par une unité de commande d'une installation (1) d'imagerie par résonnance magnétique suivant n'importe laquelle de (A) les revendications 8, 10 à 13, afin d'effectuer les stades du procédé suivant l'une quelconque des revendications 1, 3 à 7, lorsque les éléments de programme sont exécutés par l'unité de commande, ou (B) les revendications 9 à 13, lorsque les éléments de programme sont exécutés par l'unité de commande, afin d'effectuer les stades du procédé suivant l'une quelconque des revendications 2 à 7, lorsque les éléments de programme sont exécutés par l'unité d'ordinateur.

FIG 1

FIG 2

FIG 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017203330 A1